Europäisches Patentamt

European Patent Office

Office européen des brevets

(11) Publication number: **0 278 168**
**A1**

## EUROPEAN PATENT APPLICATION

(21) Application number: **87310940.9**

(22) Date of filing: **11.12.87**

(51) Int. Cl.⁴: **A61K 31/44** , A61K 9/66

The title of the invention has been amended (Guidelines for Examination in the EPO, A-III, 7.3).

(30) Priority: **12.12.86 GB 8629761**

(43) Date of publication of application:
**17.08.88 Bulletin 88/33**

(84) Designated Contracting States:
**AT CH DE FR GB IT LI NL SE**

(71) Applicant: **HARRIS PHARMACEUTICALS LTD**
**Patman House George Lane**
**South Woodford London, E18 2LS(GB)**

(72) Inventor: **Woolfe, John**
**31 Emberson Way**
**North Weald Essex CM16 6DL(GB)**

(74) Representative: **Turner, Paul Malcolm**
**URQUHART DYKES & LORD 91 Wimpole**
**Street**
**London W1M 8AH(GB)**

(54) **Nifedipine capsules.**

(57) A capsule containing one part by weight of nifedipine, 5 to 98 parts by weight of at least one polyalkylene glycol having 2 or 3 carbon atoms in the alkylene radicals and a mean molecular weight of 200 to 4000 and 0.05 to 10 parts by weight of a sorbitan ester.

EP 0 278 168 A1

## "CAPSULES"

The present invention relates to capsules and in particular to capsules for nifedipine.

GB1362627 describes a soft gelatin capsule containing a mixture of nifedipine as the active ingredient together with a polyalkylene glycol and an alcohol. At room temperature 1 part of nifedipine is soluble in 20 parts of polyethylene glycol, but that such a solution precipitates when cooled to 5°C. The addition of the alcohol, for example benzyl alcohol, is probably necessary to increase the solubility of the nifedipine, particularly at low temperatures. It is, of course, necessary in the final product to ensure that no precipitation of nifedipine occurs inside the soft capsule at any temperature normally encountered as this might adversely affect the biological absorption of the active drug. Some of the alcohols used are not in general suitable for internal use and are usually used in ointments and creams. Benzyl alcohol is a colourless aromatic liquid with a sharp burning taste, often used as a local anaesthetic. Normally other excipients are necessary, for example water, flavouring, etc. These excipients can adversely affect the solubility of nifedipine, particuarly at low temperatures.

The present invention attempts to overcome the disadvantages of the prior art and provide a carrier for the nifedipine.

The present invention provides a capsule containing one part by weight of nifedipine, 5 to 90 parts by weight of at least one polyalkylene glycol having 2 or 3 carbon atoms in the alkylene radicals and a mean molecular weight of 200 to 4000 and 0.05 to 10 parts by weight of a sorbitan ester.

The capsules are preferably gelatin capsules. Gelatin capsules are well-known and freely available commercially. The capsules may include various additives such as dyes, opacifiers, taste modifiers etc. The capsules merely contain the active ingredients of the present invention as detailed above.

It has been found surprisingly that the addition of a sorbitan ester, particularly "polysorbate", prevents the precipitation of nifedipine from solutions in a polyalkylene glycol, when these are cooled to 5°C, without the need for the addition of an alcohol.

The nifedipine is not readily dissolved in sorbitan ester. For example 5 parts of nifedipine when sonicated for 60 minutes in 100 parts of polysorbate do not dissolve. Significant heat is therefore required to effect such a solution. However, the addition of a sorbitan ester to the polyalkylene glycol and nifedipine surprisingly prevents the precipitation in the resulting solution of nifedipine on cooling. For example, 5 parts of nifedipine dissolve in 100 parts of polyethylene glycol 400 at room temperature, but precipitation occurs when the solution is cooled to 5°C and left to stand for 12 hours. Addition of 5 parts of polysorbate 80 prevents precipitation despite the fact that of itself polysorbate 80 is a poor solvent for nifedipine at 5°C.

The polyalkylene glycol is preferably present in an amount of 20 to 40 parts by weight generally about 25 parts by weight. The polyalkylene glycol is preferably polyethylene glycol having a molecular weight of 300 to 600.

The sorbitan ester may be present in an amount of 0.5 to 3 parts by weight generally about 1 part by weight. The preferred sorbitan ester also contains a polyoxyethylene chain and may contain one or more palmitic, stearic or oleic acid esters. The particularly preferred sorbitan ester is polyoxyethylene 20 sorbitan monooleate.

Particular examples of the present invention are outlined below. The following mixtures are prepared and inserted in soft gelatin capsules.

Mixtures for soft capsules can be made from a formula such as:-

EXAMPLE 1 Nifedipine     1 g
Polyethylene Glycol 400    25 g
Polysorbate 60    1 g

EXAMPLE 2 Nifedipine     1 g
Polyethylene Glycol 400    25 g
Polysorbate 80    1 g
Water    1 g
Peppermint Oil    0.001 g

## Claims

1. A capsule containing one part by weight of nifedipine, 5 to 98 parts by weight of at least one polyalkylene glycol having 2 or 3 carbon atoms in the alkylene radicals and a mean molecular weight of 200 to 4000 and 0.05 to 10 parts by weight of a sorbitan ester.

2. A capsule as claimed in claim 1 in which the sorbitan ester also contains a polyoxyethylene chain.

3. A capsule as claimed in claim 1 or claim 2 in which the sorbitan ester contains one or more palmitic, stearic or oleic acid esters.

4. A capsule as claimed in any one of the preceding claims in which the sorbitan ester is polyoxyethylene 20 Sorbitan Mono-oleate.

5. A capsule according to any one of the preceding claims in which the polyalkylene glycol is polyethylene glycol of molecular weight 300 to 600.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 93, no. 16, 20th October 1980, page 354, abstract no. 1558462z, Columbus, Ohio, US; & JP-A-80 22 631 (OHTA PHARMACEUTICAL CO., LTD) 18-02-1980 --- | 1-5 | A 61 K 31/44 A 61 K 9/66 |
| D,Y | LU-A- 65 929 (BAYER) * Page 11, examples; claims * & GB-A-1 362 627 (Cat. D,Y) --- | 1-5 | |
| Y | EP-A-0 143 857 (SIEGFRIED AG) * Page 2, line 34 - page 5, line 6; page 5, lines 30-36; claims 1,4,8 * --- | 1-5 | |
| Y | EP-A-0 117 888 (BAYER) * Page 4, line 8 - page 8, line 3; page 11, example 8; claims 1-4,6 * ----- | 1-5 | |
| | | | TECHNICAL FIELDS SEARCHED (Int. Cl.4) A 61 K |

The present search report has been drawn up for all claims

| Place of search | Date of completion of the search | Examiner |
|---|---|---|
| THE HAGUE | 22-03-1988 | BENZ K.F. |